# FASCICULE DE BREVET EUROPEEN

(11) **EP 0 549 414 B1**
(45) Date de publication et mention de la délivrance du brevet: **18.09.1996**
(21) Numéro de dépôt: 92403416.8
(22) Date de dépôt: 15.12.1992
(51) Int. Cl.: G01N 33/80, G01N 15/12

(54) **Procédé de numération des hématies ou des thrombocytes et dispositif de mise en oeuvre**
Verfahren zur Zählung von Erythrozyten oder Plättchen und Einrichtung zur Durchführung des Verfahrens
Method for enumeration of erythrocytes or platelets and device for implementing said method

(30) Priorité: 24.12.1991 FR 9116100
(43) Date de publication de la demande: 30.06.1993
(73) Titulaire: Melet, François, F-95620 Parmain (FR)
(72) Inventeur: Melet, François, F-95620 Parmain (FR)
(74) Mandataire: Rinuy, Santarelli

(56) Documents cités:
- EP-A- 0 335 789
- EP-A- 0 430 750
- FR-A- 2 551 551
- DATABASE WPI Section Ch, Week 8005, Derwent Publications Ltd., London, GB; Class B04, AN 80-08862

## Description

La présente demande concerne un procédé de numération des hématies ou des thrombocytes et son dispositif de mise en oeuvre.

Les appareils conventionnels de numération des éléments figurés du sang sont habituellement satisfaisants pour les échantillons de sang humain. On a ainsi une discrimination correcte en ce qui concerne les hématies et les thrombocytes. Il serait toutefois souhaitable d'améliorer encore cette discrimination en éliminant les possibilités d'interférence dans la numération de ces éléments.

Par ailleurs pour de nombreuses espèces d'animaux, la numération sanguine pose des problèmes. En effet, par exemple chez le chat la différenciation volumétrique des hématies et des thrombocytes est mauvaise étant donné que le volume des plus petites hématies et des plus gros thrombocytes sont comparables; de ce fait, lorsque la détection de ces éléments est réalisée selon une technique prenant en compte le volume desdits éléments telle que la numération réalisée à l'aide de compteurs automatiques utilisant le principe de mesure par impédance ou la mesure optique au laser, la numération donne des résultats erronés. Les appareils de l'état de la technique procèdent généralement à une numération de la totalité des thrombocytes et des hématies, et introduisent un seuil de détection arbitraire pour supprimer du comptage les plus gros éléments correspondant à la majeure partie, mais non la totalité, des hématies.

Il serait donc de plus souhaitable de disposer d'un procédé et d'un appareillage permettant une bonne discrimination des hématies et des thrombocytes quelle que soit la provenance de l'échantillon.

C'est pourquoi la présente demande a pour objet un procédé de numération des hématies ou des thrombocytes, caractérisé en ce que l'on effectue sur un échantillon la numération globale des thrombocytes et des hématies, ajoute au dit échantillon un agent lysant les hématies, effectue la numération des seuls thrombocytes et si désiré en déduit par simple différence le nombre d'hématies.
Par numération globale des thrombocytes et des hématies, l'on entend que l'on effectue une numération de la totalité de ces éléments sans discrimination de l'un vis-à-vis de l'autre.

Par agent lysant les hématies l'on entend tout agent capable de briser substantiellement les hématies sans être par contre agressif pour les thrombocytes. De préférence l'agent de lyse est un agent tensio-actif, notamment un émulsifiant telle qu'une saponine. On peut citer par exemple les agents de lyse commercialisés sous les noms CYMET AUTOLYSE III DIFF, LYSERGLOBINE, LYSOGLOBINE et CYMET AUTOMATED. On retient notamment celui commercialisé par la Société MELET-SCHLOESING sous le nom d'ACTILYSE^{R}.

Comme on l'a vu ci-dessus la discrimination entre hématies et thrombocytes est habituellement satisfaisante chez l'homme, c'est pourquoi le procédé ci-dessus décrit sera de préférence mis en oeuvre sur un échantillon de sang provenant d'un animal tel qu'un palmipède, un batracien, un poisson, ou de préférence un mammifère autre que l'homme. On peut citer par exemple le cheval, le chat, le chien, et de manière plus générale les animaux domestiques, de ferme, de compagnie ou de zoos.

La présente invention vise toutefois également la numération du sang humain.

La présente demande a également pour objet un dispositif de numération adapté à la mise en oeuvre du procédé ci-dessus décrit dans lequel l'on met en oeuvre l'appareillage conventionnel de numération des lignées leucocytaire, érythrocytaire, et thrombocytaire caractérisé en ce que la partie de l'appareillage concernant la numération des éléments de la lignée érythrocytaire et thrombocytaire comprend un dispositif d'arrivée dans l'échantillon d'un agent de lyse des hématies.

Un type d'appareillage conventionnel est par exemple celui décrit dans la demande de brevet européen N° 0 335 789. Un tel dispositif de numération comprend notamment un dispositif de prélèvement et de distribution de sang total et d'un liquide de dilution, un circuit de sang dilué ou de liquide de dilution, un circuit de liquide détergent pour le nettoyage des cuves et des conduits et un circuit de liquide de lyse afin de détruire spécifiquement les érythrocytes. Ces circuits comportent une cuve de dilution, une cuve de comptage des leucocytes associée à une cuve de liquide détergent, une cuve de comptage des hématies et des thrombocytes associée à une cuve de liquide détergent, une cuve de rinçage, une cuve de détermination de l'hémoglobine et une cuve de déchets mise sous dépression ou pression par une pompe pneumatique.

L'enseignement de la demande de brevet européen précitée est introduit ici par référence.

Jusqu'à présent, un éventuel agent de lyse des hématies était mis en oeuvre uniquement pour permettre la numération des leucocytes sans interférence possible des hématies. De ce fait la partie de l'appareillage concernant la numération des éléments de la lignée érythrocytaire et thrombocytaire ne comprenait aucun dispositif permettant l'adjonction d'un agent de lyse des hématies dans l'échantillon à mesurer. Au contraire on considérait qu'il était même dangereux d'amener un agent de lyse des hématies dans les canalisations et cuves permettant précisément le comptage desdites hématies.

Dans des conditions préférentielles de réalisation du dispositif de numération ci-dessus décrit, le dispositif d'arrivée dans l'échantillon d'un agent de lyse des hématies est situé dans la cuve de numération globale des hématies et des thrombocytes.

Dans un autre mode de réalisation du dispositif ci-dessus décrit, la partie de l'appareillage destinée à la numération des hématies comporte successivement deux éléments de détection et de comptage, une adjonction de produit de lyse étant réalisée entre les deux. Ainsi le premier élément effectue une numération globale des hématies et des thrombocytes alors que le second élément ne compte que les thrombocytes.

C'est, pourquoi la présente invention a ainsi pour objet un dispositif tel que ci-dessus décrit, caractérisé en ce que le dispositif d'arrivée dans l'échantillon d'un agent de lyse des hématies est situé dans la cuve de numération globale des hématies et des thrombocytes. La même cuve permet donc la mesure des hématies et des thrombocytes.

Dans encore un autre mode de réalisation, le dispositif ci-dessus décrit comporte deux circulations distinctes pour le cheminement des éléments des seules lignées érythrocytaire et thrombocytaire, l'une seule d'entre elles comportant un dispositif d'arrivée dans l'échantillon d'un agent de lyse des hématies.

C'est pourquoi la présente demande a encore pour objet un dispositif tel que ci-dessus décrit caractérisé en ce qu'il comporte deux circulations distinctes pour le cheminement des éléments des seules lignées érythrocytaire et thrombocytaire, l'une seule d'entre elles comportant un dispositif d'arrivée dans l'échantillon d'un agent de lyse des hématies.

Les exemples de réalisation qui suivent illustrent la présente invention :

### Exemple 1. Numération réalisée sur un sang de chat sain

On introduit dans une cuve de dilution environ 20 µl de sang total d'un chat, effectue une première dilution environ au 1/200 à l'aide d'environ 10 ml d'un diluant isotonique de composition suivante :

| | |
|---|---|
| EDTA | 70 g |
| K Cl | 64 g |
| Na Cl | 172 g |
| Phosphate monopotassique | 60 g |
| Phosphate disodique | 60 g |
| Bicarbonate de sodium | 18 g |
| Eau pour préparation injectable qsp | 20 L |

effectue sur cet échantillon une seconde dilution environ au 1/200, et transfère l'échantillon ainsi obtenu dans une cuve de comptage du type utilisé dans l'appareil MS 8 vétérinaire commercialisé par la Société MELET-SCHLOESING.

On envoie à l'aide d'une pompe l'échantillon dilué ci-dessus dans un élément de détection tel que ceux utilisés dans l'appareil MS 8 précité et obtient une première mesure qui est de 490 000 éléments par mm³. Il est utile à ce niveau de rappeler que cette mesure est censée correspondre à la numération classique des thrombocytes, la majeure partie des hématies ayant été électroniquement supprimée du résultat (voir notamment l'appareil MS 8 vétérinaire précité) par coupure arbitraire de la détection au delà d'un certain volume des éléments.

On rajoute alors dans la même cuve de comptage 500 µl d'agent de lyse stérile ( ACTILYSE^{R} ultrafiltrée ), on mélange et effectue une seconde mesure comme ci-dessus. On obtient la valeur de 380 000 thrombocytes réels par mm³.

### Conclusion

Une erreur d'environ 30 % est évitée sur une numération de thrombocytes dans ce cas.

### Exemple 2 : Numération réalisée sur un sang de chat atteint de pyroplasmose .

On effectue les mesures comme ci-dessus et obtient dans ce cas, pour le premier comptage 170 000 éléments par mm³, (assimilés dans l'art antérieur au thrombocytes comme on l'a vu à l'exemple 1), et 55 000 thrombocytes par mm³ pour le second comptage.

### Conclusion

Dans ce cas, l'objet de la présente demande évite une erreur de 200 % sur le comptage des thrombocytes. En effet en cas de pyroplasmose, on trouve très peu de plaquettes de faible volume ; de ce fait l'interférence est importante entre les grosses plaquettes à mesurer et les plus petites hématies.

## Revendications

1. Procédé de numération des hématies ou des thrombocytes, caractérisé en ce que l'on effectue sur un échantillon la numération globale des thrombocytes et des hématies, ajoute audit échantillon un agent lysant les hématies, effectue la numération des seuls thrombocytes et si désiré en déduit par simple différence le nombre d'hématies.

2. Procédé selon la revendication 1, caractérisé en ce que l'agent de lyse est un agent tensio-actif

3. Procédé selon la revendication 2, caractérisé en ce que le tensio-actif est une saponine.

4. Procédé selon l'une des revendications 1 à 3, caractérisé en ce que l'échantillon de sang provient d'un mammifère autre que l'homme.

5. Dispositif de numération pour la mise en oeuvre du procédé selon l'une des revendications 1 à 4 dans lequel l'on met en oeuvre l'appareillage conventionnel de numération des lignées leucocytaire, érythrocytaire, et thrombocytaire caractérisé en ce que la partie de l'appareillage concernant la numération des éléments de la lignée érythrocytaire et thrombocytaire comprend un dispositif d'arrivée dans l'échantillon d'un agent de lyse des hématies.

6. Dispositif de numération selon la revendication 5, caractérisé en ce que le dispositif d'arrivée dans l'échantillon d'un agent de lyse des hématies est situé dans la cuve de numération globale des hématies et des thrombocytes.

7. Dispositif de numération selon la revendication 5, caractérisé en ce que le dispositif d'arrivée dans l'échantillon d'un agent de lyse des hématies est situé en aval du système de numération globale des hématies et des thrombocytes.

8. Dispositif de numération selon la revendication 5, caractérisé en ce qu'il comporte deux circulations distinctes pour le cheminement des éléments des seules lignées érythrocytaire et thrombocytaire, l'une seule d'entre elles comportant un dispositif d'arrivée dans l'échantillon d'un agent de lyse des hématies.

## Patentansprüche

1. Verfahren zur Zählung der Erythrozyten oder der Thrombozyten, dadurch gekennzeichnet, daß man an einer Probe die Gesamtzählung der Thrombozyten und der Erythrozyten durchführt, der Probe ein Mittel zur Lyse der Erythrozyten hinzugibt, die Zählung nur der Thrombozyten durchführt und, falls gewünscht, die Zahl der Erythrozyten daraus durch einfache Differenzbildung ableitet.

2. Verfahren gemäß Anspruch 1, dadurch gekennzeichnet, daß das lysierende Mittel ein oberflächenaktives Mittel ist.

3. Verfahren gemäß Anspruch 2, dadurch gekennzeichnet, daß das oberflächenaktive Mittel ein Saponin ist.

4. Verfahren gemäß einem der Ansprüche 1 bis 3, dadurch gekennzeichnet, daß die Blutprobe von einem Säugetier, jedoch nicht von einem Menschen stammt.

5. Zählvorrichtung zur Durchführung des Verfahrens gemäß einem der Ansprüche 1 bis 4, in dem man die herkömmliche Zählvorrichtung für Leukozyten-, Erythrozyten- und Thrombozytenstämme benutzt, dadurch gekennzeichnet, daß der Teil des Geräts, der das Zählen der Elemente der Erythrozyten- und Thrombozytenstämme betrifft, eine Zufuhrvorrichtung für ein Mittel zur Lyse der Erythrozyten zur Probe aufweist.

6. Zählvorrichtung gemäß Anspruch 5, dadurch gekennzeichnet, daß die Zufuhrvorrichtung für ein Mittel zur Lyse der Erythrozyten zur Probe in der Küvette für die Gesamtzählung der Erythrozyten und der Thrombozyten untergebracht ist.

7. Zählvorrichtung gemäß Anspruch 5, dadurch gekennzeichnet, daß die Zufuhrvorrichtung für ein Mittel zur Lyse der Erythrozyten zur Probe in Strömungsrichtung nach dem System für die Gesamtzählung der Erythrozyten und der Thrombozyten untergebracht ist.

8. Zählvorrichtung gemäß Anspruch 5, dadurch gekennzeichnet, daß sie zwei gesonderte Kreisläufe für die Wegführung der Elemente nur der Erythrozyten- und Thrombozytenstämme aufweist, wobei nur einer die Zufuhrvorrichtung für ein Mittel zur Lyse Erythrozyten zur Probe aufweist.

## Claims

1. Process for counting erythrocytes or thrombocytes, characterized in that the overall count of thrombocytes and erythrocytes is carried out on a blood sample, an erythrocyte lysis agent is added to the said sample, a count of the thrombocytes only is taken and if desired the number of erythrocytes is deduced from the difference.

2. Process according to claim 1, characterized in that the lysis agent is a surfactant.

3. Process according to claim 2, characterized in that the surfactant is a saponin.

4. Process according to one of claims 1 to 3, characterized in that the blood sample is taken from a mammal other than man.

5. Counting device for the implementation of the process according to one of claims 1 to 4 in which the conventional equipment for counting leukocyte, erythrocyte and thrombocyte lines is used, characterized in that the part of the equipment relating to the counting of the components of the erythrocyte and thrombocyte lines contains a device for introducing an erythrocyte lysis agent into the sample.

6. Counting device according to claim 5, characterized in that the device for introducing an erythrocyte lysis agent into the sample is situated in the tank for the overall count of erythrocytes and thrombocytes.

7. Counting device according to claim 5, characterized in that the device for introducing an erythrocyte lysis agent into the sample is situated downstream of the system for the overall count of erythrocytes and thrombocytes.

8. Counting device according to claim 5, characterized in that it contains two separate circuits for the flow of components of the single erythrocyte and thrombocyte lines, only one of them containing a device for introducing an erythrocyte lysis agent into the sample.
